# EUROPÄISCHE PATENTSCHRIFT

(11) **EP 2 687 207 B1**
(45) Veröffentlichungstag und Bekanntmachung des Hinweises auf die Patenterteilung: **14.01.2015**
(21) Anmeldenummer: 11869859.6
(22) Anmeldetag: 26.07.2011
(51) Int. Cl.: A61K 31/132, A61K 33/44, B82B 1/00, B82Y 5/00, A61K 9/14, A61K 47/04, A61K 47/02, A61K 47/48, A61K 33/00

(54) **SYSTEM ZUR FREISETZUNG VON BIOLOGISCHEN WIRKSTOFFEN IN EINEM ORGANISMUS UND VERFAHREN ZUR HERSTELLUNG DES SYSTEMS**
SYSTEM FOR DELIVERING BIOLOGICALLY ACTIVE AGENTS INTO AN ORGANISM AND METHOD FOR PRODUCING SAID SYSTEM
SYSTÈME POUR L'ADMINISTRATION D'ADDITIFS BIOACTIFS DANS LE CORPS ET PROCÉDÉ DE SA FABRICATION

(43) Veröffentlichungstag der Anmeldung: 22.01.2014
(73) Patentinhaber: Zakrytoe Aktsionernoe Obschestvo "Almaz Pharm", Moscow 117393 (RU)
(72) Erfinder: YAKOVLEV, Ruslan Jur'evich, Moscow 117036 (RU)
(74) Vertreter: Jeck, Anton
(86) Internationale Anmeldenummer: PCT/RU2011/000552
(87) Internationale Veröffentlichungsnummer: WO 2013/015704

(56) Entgegenhaltungen:
- US-A1- 2009 226 495
- US-B2- 7 820 130
- US-B2- 7 820 130
- LISICHKIN G V ET AL: "Halogenation of detonation-synthesised nanodiamond surfaces", MENDELEEV COMMUNICATIONS, INSTITUTE OF PHYSICS PUBLISHING, BRISTOL, GB, Bd. 19, Nr. 6, 1. November 2009 (2009-11-01), Seiten 309-310, XP026770054, ISSN: 0959-9436 [gefunden am 2009-11-20]
- AMANDA SCHRAND ET AL: "Nanodiamond Particles: Properties and Perspectives for Bioapplications", CRITICAL REVIEWS IN SOLID STATE AND MATERIALS SCIENCES, Bd. 34, Nr. 1, 1. Januar 2009 (2009-01-01) , Seiten 18-74, XP55064590, ISSN: 1040-8436, DOI: 10.1080/10408430902831987
- ANDO ET AL: "Chemical modification of diamond surfaces using a chlorinated surface as an intermediate state", DIAMOND AND RELATED MATERIALS, ELSEVIER SCIENCE PUBLISHERS, AMSTERDAM, NL, Bd. 5, Nr. 10, 1. August 1996 (1996-08-01) , Seiten 1136-1142, XP005464376, ISSN: 0925-9635, DOI: 10.1016/0925-9635(96)00529-8
- KUANG-KAI LIU ET AL: "Covalent linkage of nanodiamond-paclitaxel for drug delivery and cancer therapy;Covalent linkage of nanodiamond-paclitaxel for drug delivery and cancer therapy", NANOTECHNOLOGY, IOP, BRISTOL, GB, Bd. 21, Nr. 31, 15. Juli 2010 (2010-07-15) , Seite 315106, XP020195971, ISSN: 0957-4484, DOI: 10.1088/0957-4484/21/31/315106
- LISICHKIN G.V.: 'Halogenation of detonation-synthesised nanodiamond surfaces' MENDELEEV COMMUN. Bd. 19, 2009, Seiten 309 - 310, XP026770054
- KULAKOVA . ET AL.: 'Stroenie chastits khimicheski modifitsirovannogo nanoalmaza detonatsionnogo sinteza' ROSSIISKIE NANOTEKHNOLOGII Bd. 5, Nr. 7-8, 2010, Seiten 66 - 73, XP008168220

## Beschreibung

Die Erfindung betrifft ein System zur Förderung der Aufnahme von bioaktiven Substanzen in den Körper und ein Herstellungsverfahren dafür.

Die Erfindung ist in der Pharmazie, der pharmazeutischen Nanotechnologie und der Pharmakologie einsetzbar. Sie betrifft ein System zur Förderung von bioaktiven Substanzen, darunter Arzneimitteln, in den Körper und ein Herstellungsverfahren dafür. Die Erfindung kann in der Medizin angewendet werden.

Aus dem Stand der Technik sind Systeme in Form von 10 - 30 nm großen Phospholipid-Nanopartikeln zur Förderung von bioaktiven Substanzen, darunter Arzneimitteln, in den Körper bekannt. Ein solches System schließt pflanzliches Phosphatidylcholin und Maltose ein [1].

Die bekannten Systeme zur Förderung von bioaktiven Substanzen, darunter Arzneimitteln, in den Körper werden in Form ihrer Kombination mit einem Polymerfüllmittel gestaltet. Die Nanopartikel enthaltenden Heilmittel können produziert werden, indem eine bioaktive Substanz oder ein Arzneimittel während oder nach der Herstellung einer Polymerdispersion eingeführt wird. Aktive Bestandteile lösen sich auf, werden aufgenommen oder auf der Oberfläche der Nanopartikel adsorbiert. Diese Mechanismen können auch kombiniert werden [2]. Jedoch können Polymer-Nanopartikel wesentliche Nachteile aufweisen. Die meisten Monomere bis auf Alkyl-Cyanoacrylate bilden biologisch schwer oder gar nicht abbaubare Polymere. Darüber hinaus gibt es keine Möglichkeit, die Molekularmasse des Polymerstoffs vollständig zu kontrollieren. Die Polymerisationsreste können giftig sein und einer nachfolgenden Reinigung des Kolloidsystems bedürfen. Während der Polymerisation können die Monomermoleküle des Öfteren mit den Arzneimittelmolekülen zusammenwirken. Das führt meistens zu ihrer Inaktivierung oder Zerstörung [3].

Durch [4] ist ein System zur Arzneimittelzufuhr mittels Nanodiamanten mit einer Korngröße von 5 nm bekannt. Dieses System umfasst ein adsorbiertes Antibiotikum Doxorubicin und hydratisierte Wassermoleküle.

Ein bekanntes System zur Förderung von Arzneimitteln mittels Nanodiamanten umfasst karboxylierte Nanodiamantenteilchen mit einer Korngröße von 3 - 5 nm. Auf der Oberfläche der Nanodiamanten werden CH₂0(CH₂)₆NH₂-Gruppen nach einer Reihe von chemischen Transformationen gepfropft. Diese Gruppen werden dabei kovalent mit antiblastomatösem DiterpenoidPaclitaxel gebunden [5].

Aus dem Stand der Technik sind auch fluormodifizierte Nanodiamantenteilchen mit einer Korngröße von 2 - 10 nm und einem Fluorgehalt bis zu 5 % (at.) bekannt [6]. Der hergestellte fluormodifizierte Nanodiamant wird zur Produktion von Konjugaten mit solchen Substanzen wie Lithiumalkyl-Verbindungen, Diaminen und Aminosäuren verwendet. Die genannten Konjugate können als Bindemittel für Polymerzusammensetzungen, Schleifmaterial und Beschichtungen, Adsorptionsmittel, Biosensoren und nanoelektromechanischen Systeme angewendet werden.

Aus [7] ist ein Verfahren zur Erhöhung der Wirksamkeit der Arzneimittel durch chemische (kovalente) Bindung der Arzneimittelmoleküle an die Nanodiamantenteilchen mit einer Korngröße von unter 10 nm auf deren Oberfläche bekannt. Diese Bindung erfolgt mittels Fluoratomen und/oder OH- Gruppen.

Die in einem organischen Stoff vorhandenen Fluoratome steigern seine Giftigkeit. So kann z. B. ein solcher Stoff Nervensystem-, Lungen- und Leberschäden hervorrufen. Sogar perfluorierte organische Stoffe verändern trotz ihrer chemischen Inaktivität die Kennziffern des mikrosomalen Systems bei einer biologischen Transformation der Xenobiotika (lebensfremde Stoffe) in der Leber [8]. So ist z. B. nachgewiesen worden, dass kovalente Bindung der Fluoratome an das Fullerenmolekül C₆₀ seine Gesamttoxizität um das 2,4- bis 5-fache steigert. Fulleren ist die nächste nanostrukturmäßige Kohlenstoff-Entsprechung von Nanodiamanten [9].

Deswegen ist die Herstellung von fluorfreien Nanodiamantenteilchen, um diese als Systeme für die Zufuhr von bioaktiven Substanzen in den Körper nutzen zu können, eine brisante und maßgebliche Aufgabe sowohl für die Medizin als auch für die pharmazeutische Industrie.

Aus dem Stand der Technik ist ein Verfahren zur Effektivitätssteigerung der Arzneimittel mittels chemischer (kovalenter) Bindung der Arzneimittelmoleküle an die Nanodiamantenteilchen mit einer Korngröße von unter 10 nm durch Amino- oder Acylchloridgruppen auf deren Oberfläche bekannt [10].

Aus dem Stand der Technik sind auch chlormodifizierte Nanodiamantenteilchen bekannt. Hier beträgt der Chloranteil bis zu 12 % (at.). Die Korngröße der Partikel in der Suspension ist jeweils 70 nm ein Monat nach der Synthese und 180 nm 9 Monate nach der Synthese [11]. Diese Partikel sind größer als es für medizinische Zwecke optimal nötig ist. Dabei wurde kein maximaler Anteil der Chloratome auf der Oberfläche des Nanodiamanten erreicht. Folglich wird es nicht mehr möglich sein, den Grenzanteil des Arzneimittels auf der Nanodiamantenoberfläche zu bekommen. Dies vermindert offenkundig die Effektivität des Förderungssystems. Die Verfasser von [11] weisen zwar darauf hin, dass die Analyse des chlormodifizierten Nanodiamanten nach dem Verfahren der Röntgen-Photoelektronenspektroskopie die Bindung der Chloratome an die Oberflächen-Kohlenstoffatome bestätigt, jedoch werden keine Nachweisdaten beigefügt. Darüber hinaus lässt die von den Verfassern selbst vorgenommene Analyse der im Schriftstück angeführten IR-Spektren keine solchen chemischen Bindungen erkennen. Folglich bietet sich die Annahme an, dass die Chloratome auf der Nanodiamantenoberfläche adsorptionsgestützt und nicht kovalent gebunden werden. Folglich werden die Arzneimittel mit einer solchen Oberfläche des Nanodiamanten nicht fest genug chemisch gebunden, und das Förderungssystem ist dadurch nicht mehr wirksam.

[11] beschreibt ein Verfahren zur Herstellung von solchen chlormodifizierten Nanodiamantenteilchen und seine Ausgestaltung. Die Nanodiamantenteilchen werden nach dem Verfahren einer Flüssigphasenchlorierung des reduzierten Nanodiamanten in einer chlorgesättigten Lösung CCl₄ bei Raumtemperatur und unter ständigem Umrühren im Laufe von 72 Stunden unter Lichtbestrahlung im sichtbaren Bereich chloriert. Nach der Chlorierung werden die Nanodiamantenteilchen mit trockenem CCl₄ gewaschen und zentrifugiert. Der Absatz wird 5 bis 6 Stunden lang bei einem Druck von 13 - 26 Pa und einer Temperatur von 70 - 80° C getrocknet.

Eine Ausgestaltung dieses Verfahrens zur Herstellung von chlormodifizierten Nanodiamantenteilchen besteht darin, dass die Chlorierung im Plasma von CCl₄ im Laufe von 6 Stunden durchgeführt wird [11].

Die Verfasser von [11] kommen zu dem Schluss, dass die von ihnen erreichte Bindung zwischen den Chloratomen und den Nanodiamanten (wegen vermutlicher adsorptionsgestützter Herkunft der Bindung) in der Luft weniger stabil ist als die Bindung zwischen den Nanodiamanten und den Fluoratomen. Dabei überschreitet die eventuell mögliche Menge der an die Nanodiamantenoberfläche gebundenen Fluoratome die der Chloratome. Somit sind die chlorierten Nanodiamantenteilchen für weitere Reaktionen zwecks kovalenter Bindung von chemischen Verbindungen im Vergleich zu den fluorierten Nanodiamantenteilchen weniger geeignet.

Es ist Aufgabe der Erfindung, fluorfreie Nanodiamantenteilchen herzustellen, welche dazu fähig sind, verschiedene biologisch aktive Substanzen, darunter auch Arzneimittel, effektvoll kovalent zu binden. Darüber hinaus sollen die vorhandenen kovalent auf der Oberfläche der Nanodiamanten gebundenen Chloratome nachfolgend durch Moleküle von bioaktiven Substanzen völlig ersetzt werden. Dadurch wird es möglich, aussichtsreiche Systeme zur Förderung der Aufnahme von bioaktiven Substanzen in den Körper zu bekommen. Diese Systeme weisen dann keine Halogenatome auf ihrer Oberfläche auf. Dadurch wird eine unkontrollierte Steigerung der toxischen Eigenschaften vermieden. Diese Forderung ist für beliebige Arzneimittel sowie medizinische Er-zeugnisse, die in der Medizin und in der pharmazeutischen Industrie angewendet werden, von äußerster Wichtigkeit.

Die gestellte Aufgabe wird durch die Merkmale des Anspruchs 1 gelöst.

Bei dem erfindungsgemäßen System zur Förderung der Aufnahme von bioaktiven Substanzen in den Körper handelt es sich um ein graues ultradisperses Nanodiamanten-Pulver (Fig. 1) mit einer Korngröße von 2 - 10 nm (Fig. 2), mit einer chlormodifizierten Oberfläche der Nanodiamantenteilchen bei einem Chlorgehalt bis zu 14 % (at.) (Fig. 3). Die Aggregatgrößenverteilung bei dem neuen Förderungssystem beträgt 40 - 70 nm in wässriger Suspension (Fig. 4).

Die Erfindung wird anhand der beiliegenden Zeichnungen näher erläutert.

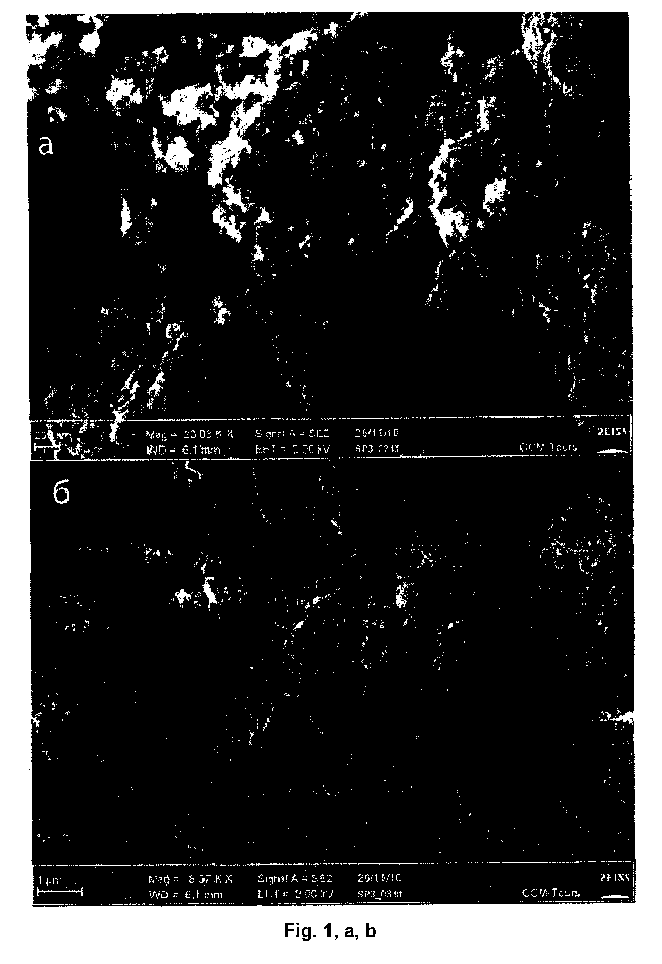

In Fig. 1 ist deutlich erkennbar, dass das neue Förderungssystem ein ultradisperses Gefüge aufweist, dessen Korngröße die Auflöseempfindlichkeit des benutzten Geräts unterschreitet (also unter 20 nm liegt).

Die Mikroaufnahmen wurden mit einem elektronischen höchstauflösenden Abtast-Feldemissionsmikroskop Zeiss Ultra Plus (Carl Zeiss, Deutschland) gemacht. Die Aufnahmebedingungen sind den Mikroaufnahmen zu entnehmen.

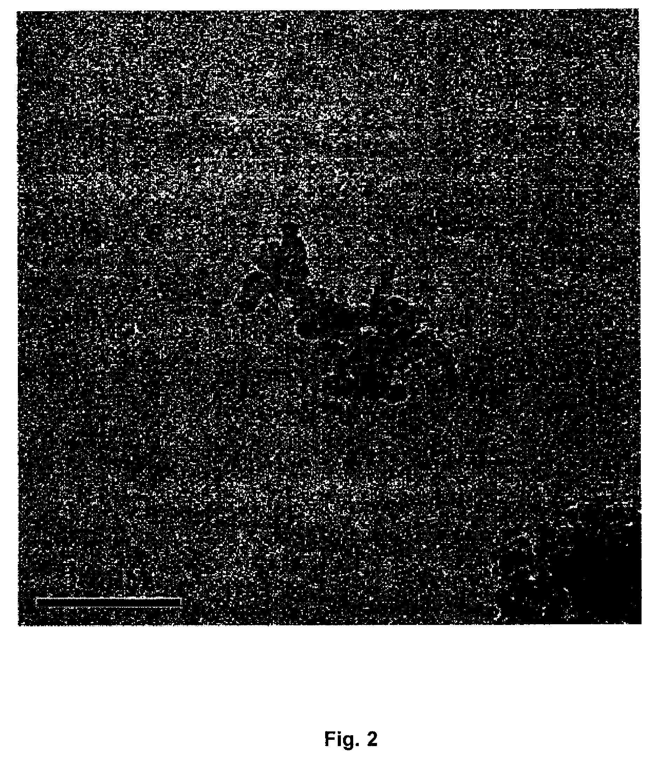

Aus Fig. 2 ist ersichtlich, dass das neue System zur Förderung von bioaktiven Substanzen eine Teilchengrößenverteilung von 2 - 10 nm aufweist.

Die Mikroaufnahme wurde mit einem Durchstrahlungselektronenmikroskop Jeol 1011 (JEOL, Japan) gemacht.

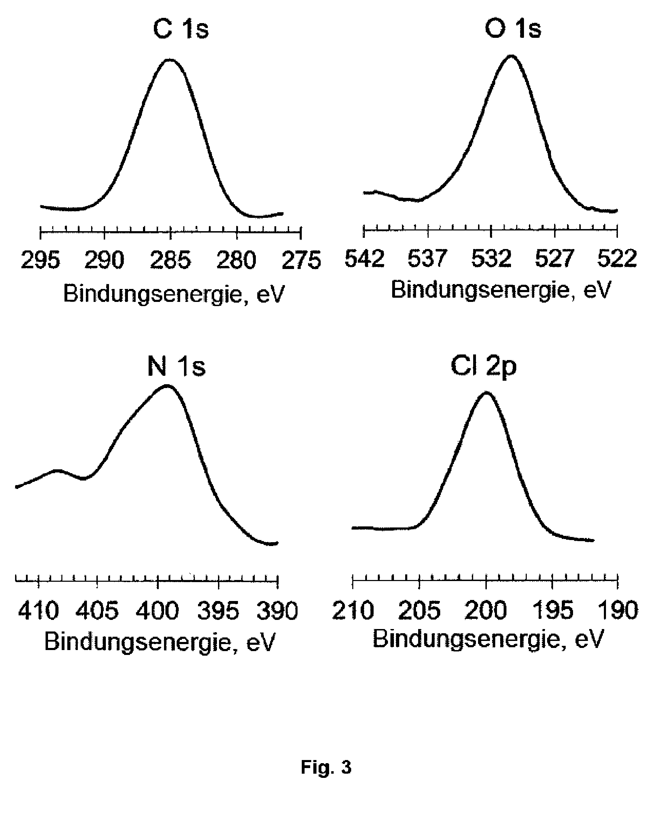

Fig. 3 zeigt Spektren der Röntgen-Photoelektronenspektroskopie für das neue System zur Förderung von bioaktiven Substanzen. Diese Spektren bestimmen den Charakter, den Energiezustand und die Menge von Oberflächenatomen der Nanodiamantenteilchen.

Die Oberfläche des neuen Systems zur Förderung von bioaktiven Substanzen wurde mit Hilfe eines LAS-3000-Geräts (Riber, Frankreich) erforscht. Das Gerät war mit einem halbkugelförmigen Analysengerät OPX-150 ausgerüstet. Die Fotoelektronen wurden mittels nichtmonochromatischer Röntgenstrahlung einer Alu-Anode (AlK_{α} = 1486,6 eV) bei einer Röhrenspannung von 12 kV und einem Emissionsstrom von 20 mA angeregt. Die Kalibrierung der Photoelektronen-Spitzenwerte wurde anhand der Kohlenstoffkurve C 1s mit einer Bindungsenergie (E_{Bind}) von 285 eV vorgenommen. Das Vakuum in der Arbeitskammer betrug 6,7·10⁻⁸ Pa. Um ein Hochvakuum aufzubauen, wurde eine Ionenpumpe verwendet.

Die Elementenzusammensetzung der Oberfläche des Systems zur Förderung von bioaktiven Substanzen nach Angaben der Röntgen-Photoelektronenspektroskopie ist der Tabelle 1 zu entnehmen.

**Tabelle 1**

| Elementenzusammensetzung und Bindungsenergien der Oberflächenatome des neuen Systems zur Förderung von bioaktiven Substanzen | | | | |
|---|---|---|---|---|
| Eigenschaft | Chemische Elemente | | | |
| | C | O | N | Cl |
| Atom %, | 76,7-91,2 | 5,0-7,1 | 1,8-2,2 | 2-14 |
| Bindungsenergien, eV | 285,3±0,5 | 530,7±0,5 | 399,5±0,5 | 200,3±0,5 |
| | | | 408,7±0,5 | |

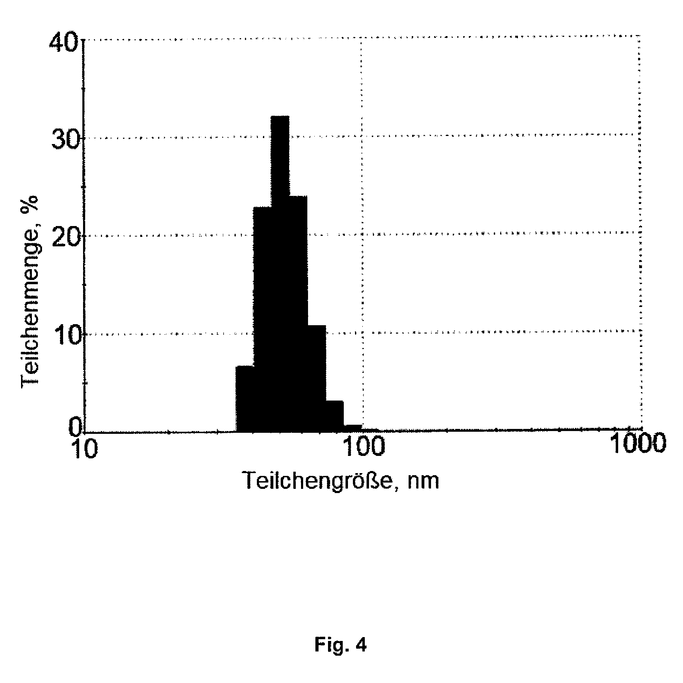

Fig. 4. zeigt eine Verteilkurve des neuen Systems zur Förderung von bioaktiven Substanzen in wässriger Suspension bei Aggregatgrößen von 40 - 70 nm.

Die Teilchengrößenverteilung des neuen Förderungssystems in der Suspension wurde nach dem Verfahren der dynamischen Laserlichtstreuung mit einem ZetaSizer-Gerät (Malvern Instruments, USA) gemessen.

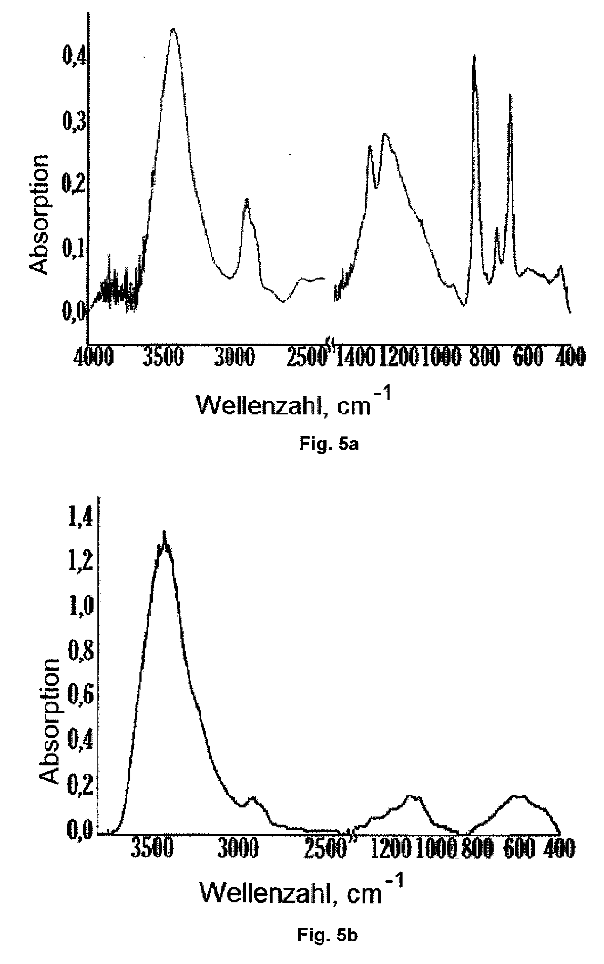

Der Fig. 5a ist das IR-Spektrum des neuen Systems zur Förderung von bioaktiven Substanzen mit einem Oberflächen-Chloranteil von 14 % (at.) zu entnehmen. Auf dem Spektrum ist Folgendes erkennbar: ein starker breiter Streifen mit einem Maximum bei 3430 cm⁻¹, ein breiter Streifen mit einem Maximum bei 1262 cm⁻¹, fünf mittelstarke Streifen bei 2929, 2892, 1331, 846, 680 cm⁻¹ und ein schwaches Signal bei 743 cm⁻¹. Dieses Spektrum zeigt, dass die Oberfläche des neuen Förderungssystems kovalent gebundene Chloratome aufweist. Ihre Valenz-Kennfrequenzen liegen im Bereich 650 - 850 cm⁻¹ [12]. Die Fig. 5b weist das IR-Spektrum des neuen Systems zur Förderung von bioaktiven Substanzen mit einem Mindestchloranteil auf der Oberfläche (0,1 % at.) auf. Auf dem Spektrum ist Folgendes erkennbar: ein starker breiter Streifen mit einem Maximum bei 3430 cm⁻¹, zwei breite Streifen mit einem Maximum bei 1136 und 621 cm⁻¹, zwei mittelstarke Streifen bei 2929, 2892 cm⁻¹ und ein schwaches Signal bei 1331 cm⁻¹. Bei einer solchen geringen Chlorkonzentration auf der Oberfläche des neuen Systems zur Förderung von bio-aktiven Substanzen lässt sich Chlor im IR-Spektrum im Bereich von 650 - 850 cm⁻¹ nicht erkennen.

Die IR-Spektren wurden mit dem FTIRS IR200 Thermonicolet Gerät (Thermo Scientific, USA) erfasst. Die Auflösung betrug 2 cm⁻¹ und die Scan-Menge 64. Die Einwaagen der Proben wurden zwecks Analyse mit KBr-Pulver vermengt und zu einer Tablette gepresst.

Das hergestellte System weist weder für den menschlichen Körper noch für Tiere gesundheitsschädliches Fluor noch Verbindungen auf, welche nach erfolgten Bindungsreaktionen zwischen den bioaktiven Substanzen und der Nanodiamantenoberfläche zurückbleiben. Deswegen kann dieses System effektvoll benutzt werden, um biologisch aktive Substanzen, darunter auch Arzneimittel, in den Körper des Menschen zu liefern.

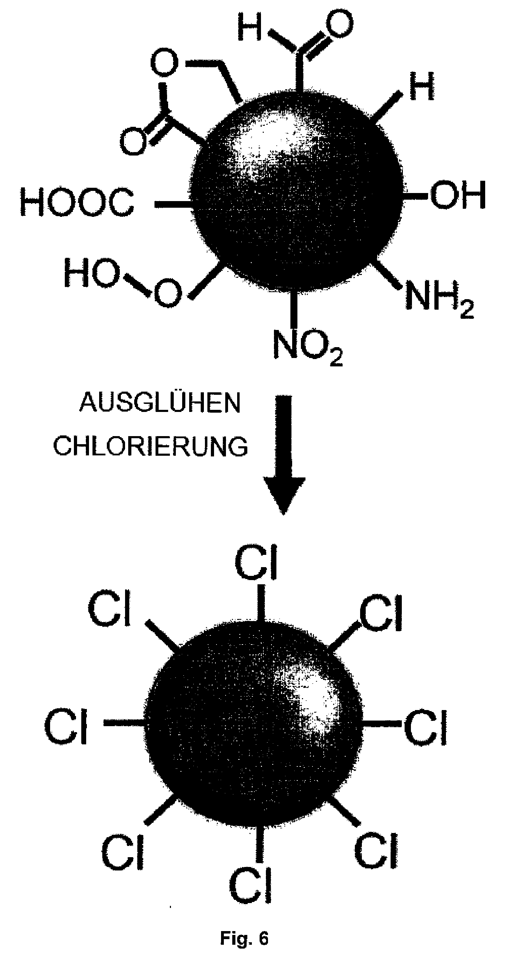

Gemäß der Erfindung wird auch ein Verfahren zur Herstellung des Systems zur Förderung von bioaktiven Substanzen beansprucht. Dieses System ist skizzenhaft in Fig. 6 dargestellt.

Das erfindungsgemäße Verfahren zur Herstellung des Systems zur Förderung der Aufnahme von bioaktiven Substanzen in den Körper wird wie folgt ausgeführt. Die Nanodiamantenteilchen werden bei einer Temperatur von 500 bis 1200° C in einer Wasserstoffgasströmung ausgeglüht. Die hergestellten ausgeglühten Nanodiamantenteilchen werden nachher mit einem in CCl₄ aufgelösten molekularen Chlor unter sichtbarem Licht bei einer Temperatur von 50 bis 70° C chloriert. Der Wasserstoffgasdurchsatz beträgt beim Ausglühen 2 bis 3 L/Stunde. Die Chlorierung dauert vorwiegend 36 bis 60 Stunden lang bei einer Chlorkonzentration CCl₄ von 3 bis 5 % (Gew.). Danach werden die Nanodiamantenteilchen zentrifugiert, mit CCl₄ gewaschen und getrocknet.

### Ausführliche Beschreibung des Verfahrens

Der Nanodiamant wird in der Wasserstoffgasströmung bei einem Wasserstoffgasdurchsatz von 2 - 3 L/Stunde und bei einer Temperatur von 500 bis 1200° C im Laufe von 1 bis 8 Stunden ausgeglüht. Danach werden die ausgeglühten Teilchen der Nanodiamanten mittels molekularen Chlors der Flüssigphasenchlorierung aus-gesetzt. Das Chlor wird dafür zuerst infolge der Reaktion zwischen K₂Cr₂O₇ (bzw. KMnO₄) und Salzsäure produziert und dann in CCl₄ aufgelöst, bis eine Konzentration von 3 - 5 % (Gew.) erreicht wird. Die Chlorierungsreaktion wird unter photochemischer Wirkung des sichtbaren Lichts im Laufe von 36 bis 60 Stunden und bei einer Temperatur von 50 - 70° C durchgeführt. Danach wird die Suspension mit einer Geschwindigkeit von über 6000 U/min. zentrifugiert und mit CCl₄ gespült. Der Vorgang wird 3 - 5-mal wiederholt. Anschließend erfolgt eine Vakuumtrocknung bis zu einem konstanten Gewicht.

Auf der Grundlage des hergestellten Förderungssystems werden Konjugate mit biologisch aktiven Substanzen, darunter Arzneimitteln, aus verschiedenen pharmakologischen Gruppen zubereitet: Alkylierungsmittel und insbesondere äthylendiaminhaltige Alkylierungsmittel, Hilfsstoffe, Reaktionsmittel und Zwischenprodukte sowie Aminosäuren.

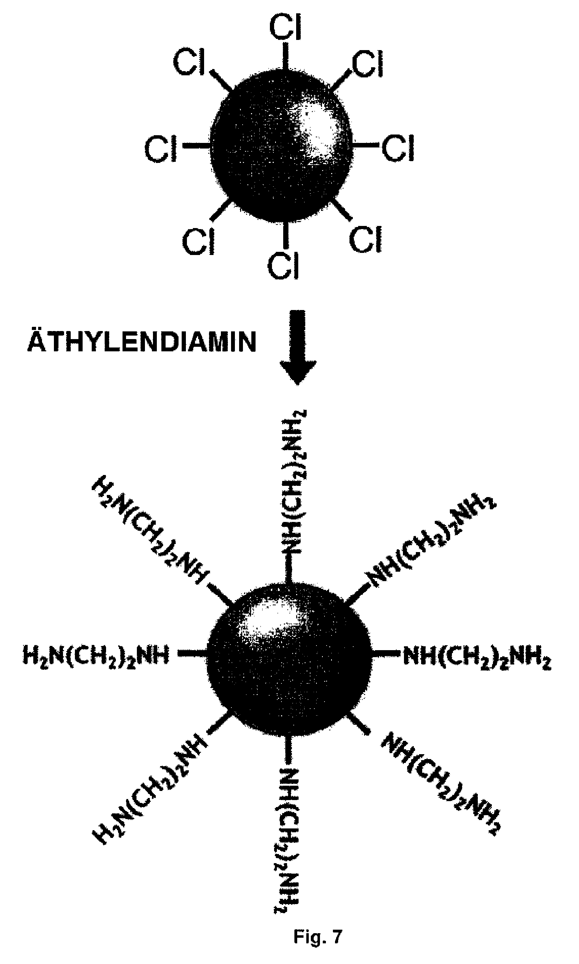

Bei Diaminen werden die hergestellten Teilchen des neuen Förderungssystems in Dimethylsulfoxid (CH₃)₂SO aufgeschwemmt, mit Äthylendiamin ergänzt, einige Tropfen Pyridin dazugegeben und bei einer Temperatur von 120° C im Laufe von 24 Stunden gehalten [Fig. 7]. Danach wird das hergestellte Konjugat von Nanodiamanten mit Äthylendiamin bei einer Geschwindigkeit von über 6000 U/min. zentrifugiert, mehrfach mit Wasser und Azeton gewaschen und anschließend im Vakuum bis zu einem konstanten Gewicht getrocknet.

Das hergestellte Konjugat dient der Zufuhr von Äthylendiamin in den Körper. Um die Lösung der gestellten Aufgabe bei der Herstellung von einem System zur Förderung der Aufnahme von bioaktiven Substanzen in den Körper nachzuweisen, wurde Nanodiamant nach dem Ausglühen mit einem Tritiummarker nach dem Verfahren einer thermischen Aktivierung durch Tritium [13] versehen. Danach entsteht ein Förderungssystem für bioaktive Substanzen gemäß dem neuen Verfahren. Die Oberfläche dieses Systems ist mit einem radioaktiven Marker markiert. Danach wird nach dem oben beschriebenen Verfahren ein Konjugat des Förderungssystems mit Äthylendiamin hergestellt. Das hergestellte Konjugat mit dem radioaktiven Marker wird in den Körper einer Ratte intraperitoneal eingeführt. Das Tier wird getötet. Die Organe werden entnommen und homogenisiert. Nachher wird die Strahlungsaktivität des erzeugten Homogenats im Flüssigkeitsszintillationsspektrometer gemessen.

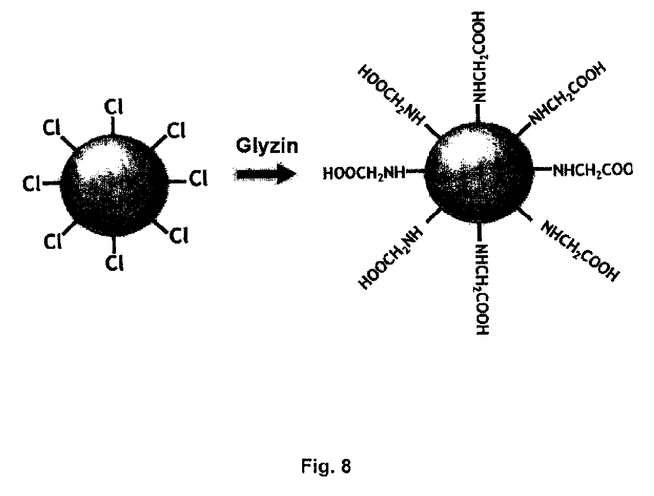

Bei Aminosäuren, z. B. Glyzin, wird sein Konjugat mit dem Förderungssystem wie folgt hergestellt (Fig. 8). Die hergestellten Teilchen des Förderungssystems werden in einem wasserorganischen Lösemittel oder im Wasser aufgelöst. Der erzeugten Suspension wird Glyzin in Form von Aminoessigsäure NH₂CH₂COOH mit Zugabe von tertiärem Amin zugesetzt. Als organisches Lösemittel sollten hauptsächlich solche Lösemittel verwendet werden, die Glyzin auflösen können, z. B. Pyridin oder niedere aliphatische Alkohole. Das hergestellte Gemisch wird mit Ultraschall (50 W) im Laufe von 5 - 60 Min. behandelt und unter ständigem Umrühren und bei einer Temperatur von 50 - 80° C innerhalb von 12 - 48 Stunden gehalten. Das erzeugte Produkt wird bei einer Geschwindigkeit von 6000 U/min. zentrifugiert und mit Äthanol gespült. Der Absatz wird im Vakuum bei 70° C über die ganze Nacht getrocknet.

Das hergestellte Konjugat wird für Glyzinlieferung in den Körper angewendet. Zu diesem Zweck wird die Zusammenwirkung des hergestellten Konjugats mit Zellkulturen nach zellbiologischen Verfahren mit Hilfe eines Elektronenmikroskops erforscht.

### Kurze Beschreibung der Grafiken

Fig. 1. Mikroaufnahmen des ultradispersen Gefüges des Systems zur Förderung von bioaktiven Substanzen. Die Mikroaufnahmen wurden nach dem Verfahren der Rasterelektronenmikroskopie gemacht (a - 23,83 Tausend x Vergrößerung; b - 8,57 Tausend x Vergrößerung),
Fig. 2. Mikroaufnahme der Teilchen des Systems zur Förderung von bioaktiven Substanzen (Durchstrahlungselektronenmikroskopie),
Fig. 3. C 1s, O 1s, N 1s, Cl 2p Spektren der Teilchenoberfläche des Systems zur Förderung von bioaktiven Substanzen (Röntgen-Photoelektronenspektroskopie),
Fig. 4. Teilchengrößenverteilung des Systems zur Förderung von bioaktiven Substanzen in wässriger Suspension (dynamische Laserlichtstreuung),
Fig. 5. IR-Spektrum des Systems zur Förderung von bioaktiven Substanzen,
Fig. 6. Herstellung des Systems zur Förderung von bioaktiven Substanzen (schematische Darstellung),
Fig. 7. Herstellung von Konjugat von Nanodiamanten mit Äthylendiamin (schematische Darstellung),
Fig. 8. Herstellung von Konjugat von Nanodiamanten mit Glyzin (schematische Darstellung),

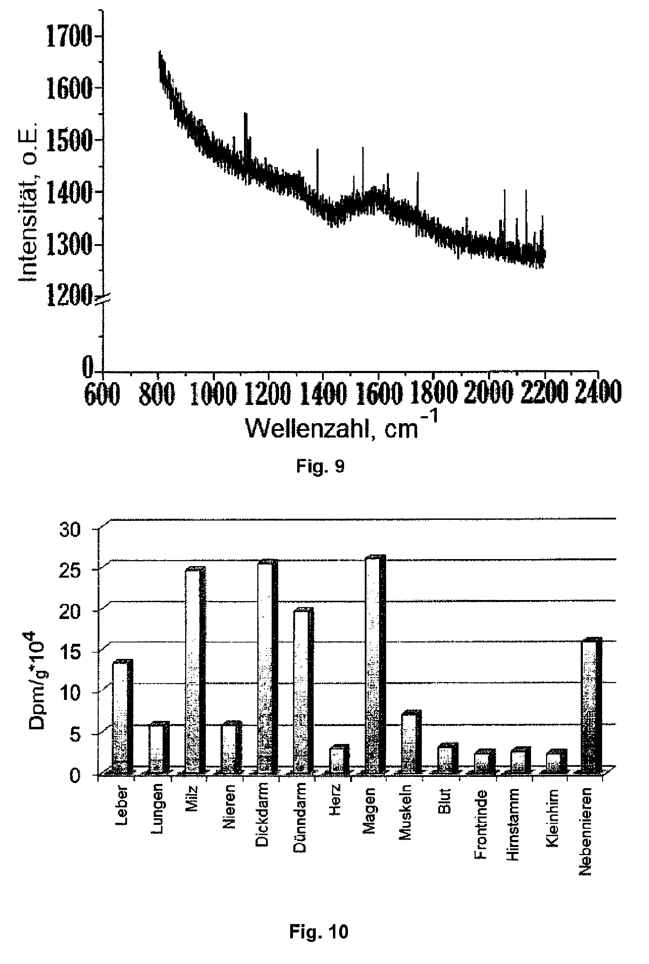

Fig. 9. Raman-Spektrum von Nanodiamanten-Konjugat mit Äthylendiamin,
Fig. 10. Bioverteilung von Nanodiamanten-Konjugat mit Äthylendiamin im Rattenkörper,

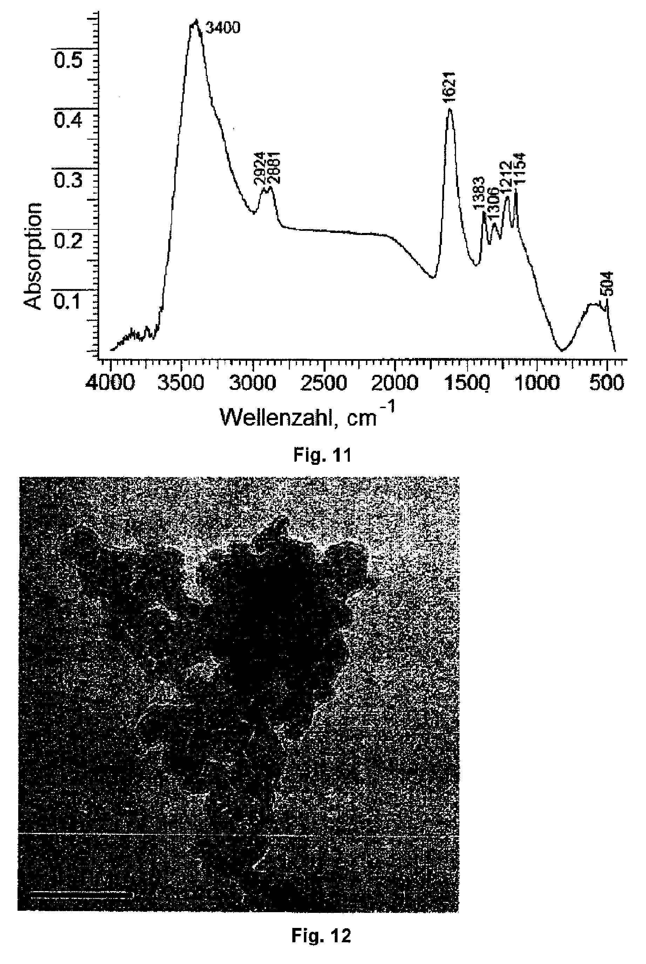

Fig. 11. IR-Spektrum von Nanodiamanten-Konjugat mit Glyzin,
Fig. 12. Mikroaufnahme von Nanodiamanten-Konjugat mit Glyzin (Durchstrahlungselektronenmikroskopie),

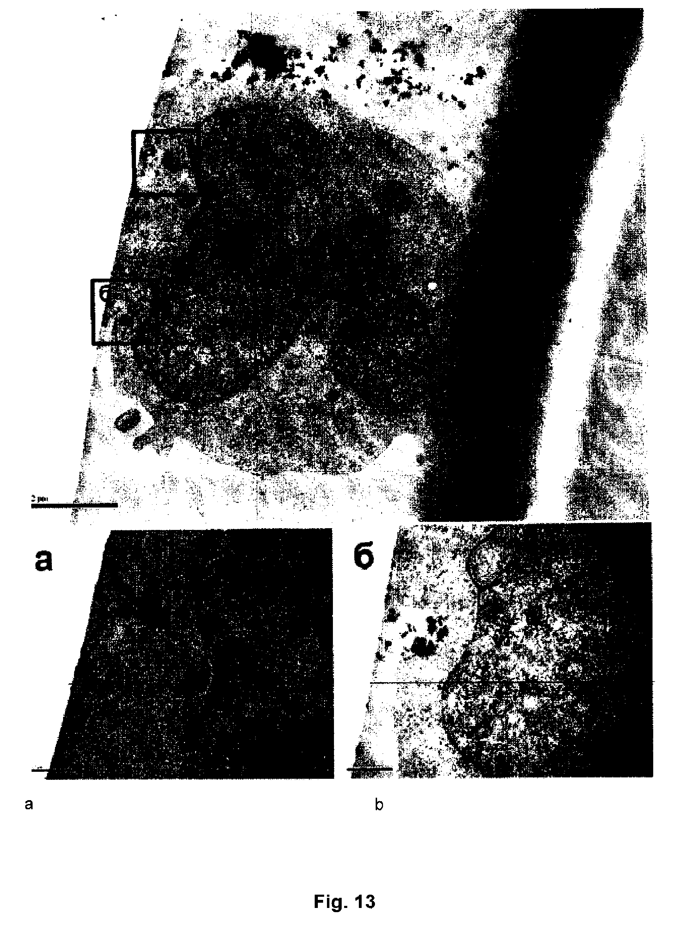

Fig. 13. Mikroaufnahme der Eindringung von Nanodiamanten-Konjugat mit Glyzin in die Lymphoblastzelle MOLT-4. a, b (markiert sind Bereiche der Eindringung der Teilchen in die Zelle).

Die Erfindung wird anhand folgender Beispiele geschildert.

### Beispiel 1

200 mg Nanodiamanten-Einwaage wird in einer Wasserstoffgasströmung mit einem Durchsatz von 2,5 L/Stunde bei 800° C im Laufe von 5 Stunden ausgeglüht. Die ausgeglühten Nanodiamantenteilchen werden nach dem Verfahren einer Flüssigphasenchlorierung mit molekularem Chlor (4,7 % (Gew.)) in 40 mL CCl₄ im sichtbaren Licht innerhalb von 48 Stunden bei einer Temperatur von 60° C chloriert. Danach wird die Suspension mit einer Geschwindigkeit von 8000 U/min. zentrifugiert und mit trockenem CCl₄ gespült. Der Vorgang wird viermal wiederholt. Der hergestellte Absatz wird anschließend im Vakuum bis zu einem konstanten Gewicht getrocknet. Die Zielproduktausbeute beträgt 181 mg (90,5 %).

Das erzeugte Produkt ist ein graues ultradisperses Pulver mit einer Korngröße von 2 - 10 nm. Der Chloranteil auf seiner Oberfläche beträgt 14 % (at.) mit Aggregatgrößen von 50 nm in wässriger Suspension. Dieses Pulver ist durch folgendes IR-Spektrum gekennzeichnet: ein starker breiter Streifen mit einem Maximum bei 3430 cm⁻¹ ein breiter Streifen mit einem Maximum bei 1262 cm⁻¹, fünf mittelstarke Streifen bei 2929, 2892, 1331, 846, 680 cm⁻¹ und ein schwaches Signal bei 743 cm⁻¹. Die Elementenzusammensetzung der Oberfläche umfasst jeweils C - 78,1, O - 6,0, N - 1,9, Cl - 14 % (at.).

### Beispiel 2

250 mg Nanodiamanten-Einwaage wird in einer Wasserstoffgasströmung mit einem Durchsatz von 2,4 L/Stunde bei 800° C im Laufe von 5 Stunden ausgeglüht. Die ausgeglühten Nanodiamantenteilchen werden nach dem Verfahren einer Flüssigphasenchlorierung mit molekularem Chlor (4,8 % Gew.) in 50 mL CCl₄ im sichtbaren Licht im Laufe von 36 Stunden bei einer Temperatur von 60° C chloriert. Danach wird die Suspension mit einer Geschwindigkeit von 8000 U/min. zentrifugiert und mit trockenem CCl₄ gespült. Der Vorgang wird 3mal wiederholt. Der hergestellte Absatz wird anschließend im Vakuum bis zu einem konstanten Gewicht getrocknet. Die Zielproduktausbeute beträgt 198 mg (79,1 %).

Das erzeugte Produkt ist ein graues ultradisperses Pulver mit einer Korngröße von 2 - 10 nm. Der Chloranteil auf seiner Oberfläche beträgt 4,2 % (at.) mit Aggregatgrößen von 67 nm in wässriger Suspension. Dieses Pulver ist durch folgendes IR-Spektrum gekennzeichnet: ein starker breiter Streifen mit einem Maximum bei 3430 cm⁻¹, ein breiter Streifen mit einem Maximum bei 1262 cm⁻¹, fünf mittelstarke Streifen bei 2929, 2892, 1331, 846, 680 cm⁻¹ und ein schwaches Signal bei 743 cm⁻¹. Die Elementenzusammensetzung der Oberfläche umfasst jeweils C - 87,9, O - 5,9, N - 2,0, Cl - 4,2 % (at.).

### Beispiel 3

400 mg Nanodiamanten-Einwaage wird in einer Wasserstoffgasströmung mit einem Durchsatz von 2,7 L/Stunde bei einer Temperatur von 800° C im Laufe von 5 Stunden ausgeglüht. Die ausgeglühten Nanodiamantenteilchen werden nach dem Verfahren einer Flüssigphasenchlorierung mit molekularem Chlor (3,5 % (Gew.)) in 80 mL CCl₄ im sichtbaren Licht 60 Stunden lang bei einer Temperatur von 60° C chloriert. Danach wird die Suspension mit einer Geschwindigkeit von 7000 U/min. zentrifugiert und mit trockenem CCl₄ gespült. Der Vorgang wird mal wiederholt. Der hergestellte Absatz wird anschließend im Vakuum bis zu einem konstanten Gewicht getrocknet. Die Zielproduktausbeute beträgt 339,6 mg (84,9 %).

Das erzeugte Produkt stellt ein graues ultradisperses Pulver mit einer Korngröße von 2 - 10 nm dar. Der Chloranteil auf seiner Oberfläche beträgt 7,8 % (at.) mit Aggregatgrößen von 56 nm in wässriger Suspension. Es ist durch folgendes IR-Spektrum gekennzeichnet: ein starker breiter Streifen mit einem Maximum bei 3430 cm⁻¹, ein breiter Streifen mit einem Maximum bei 1262 cm⁻¹, fünf mittelstarke Streifen bei 2929, 2892, 1331, 846, 680 cm⁻¹ und ein schwaches Signal bei 743 cm⁻¹. Die Elementenzusammensetzung der Oberfläche umfasst jeweils C - 84,1, O - 6,3, N - 1,8, Cl - 7,8 %) (at.).

### Beispiel 4

200 mg Nanodiamanten-Einwaage wird in einer Wasserstoffgasströmung mit einem Durchsatz von 2,0 L/Stunde bei einer Temperatur von 800° C im Laufe von 5 Stunden ausgeglüht. Die ausgeglühten Nanodiamantenteilchen werden nach dem Verfahren einer Flüssigphasenchlorierung mit molekularem Chlor (5,0 % (Gew.)) in 40 mL CCl₄ im sichtbaren Licht innerhalb von 48 Stunden bei einer Temperatur von 50° C chloriert. Danach wird die Suspension mit einer Geschwindigkeit von 6000 U/min. zentrifugiert und mit trockenem CCl₄ gespült. Der Vorgang wird 5mal wiederholt. Der hergestellte Absatz wird anschließend im Vakuum bis zu einem konstanten Gewicht getrocknet. Die Zielproduktausbeute beträgt 149,2 mg (74,6 %).

Das erzeugte Produkt ist ein graues ultradisperses Pulver mit einer Korngröße von 2 - 10 nm. Der Chloranteil auf seiner Oberfläche beträgt 3,0 % (at.) mit Aggregatgrößen von 70 nm in wässriger Suspension. Es ist durch folgendes IR-Spektrum gekennzeichnet: ein starker breiter Streifen mit einem Maximum bei 3430 cm⁻¹, ein breiter Streifen mit einem Maximum bei 1262 cm⁻¹, fünf mittelstarke Streifen bei 2929, 2892, 1331, 846, 680 cm⁻¹ und ein schwaches Signal bei 743 cm⁻¹. Die Elementenzusammensetzung der Oberfläche umfasst jeweils C - 87,8, O - 7,1, N - 2,1, Cl - 3,0 % (at.).

### Beispiel 5

200 mg Nanodiamanten-Einwaage wird in einer Wasserstoffgasströmung mit einem Durchsatz von 2,9 L/Stunde bei 800° C im Laufe von 5 Stunden ausgeglüht. Die ausgeglühten Nanodiamantenteilchen werden nach dem Verfahren einer Flüssigphasenchlorierung mit molekularem Chlor (5,0 % (Gew.)) in 40 mL CCl₄ im sichtbaren Licht innerhalb von 48 Stunden bei einer Temperatur von 70° C chloriert. Danach wird die Suspension mit einer Geschwindigkeit von 9000 U/min. zentrifugiert und mit trockenem CCl₄ gespült. Der Vorgang wird 3mal wiederholt. Der hergestellte Absatz wird anschließend im Vakuum bis zu einem konstanten Gewicht getrocknet. Die Zielproduktausbeute beträgt 144,6 mg (72,3 %).

Das erzeugte Produkt ist ein graues ultradisperses Pulver mit einer Korngröße von 2 - 10 nm. Der Chloranteil auf seiner Oberfläche beträgt 9,4 % (at.) mit Aggregatgrößen von 61 nm in wässriger Suspension. Das Produkt ist durch folgendes IR-Spektrum gekennzeichnet: ein starker breiter Streifen mit einem Maximum bei 3430 cm⁻¹, ein breiter Streifen mit einem Maximum bei 1262 cm⁻¹, fünf mittelstarke Streifen bei 2929, 2892, 1331, 846, 680 cm⁻¹ und ein schwaches Signal bei 743 cm⁻¹. Die Elementenzusammensetzung der Oberfläche umfasst jeweils C - 83,3, O - 5,5, N - 1,8, Cl - 9,4 % (at.).

### Beispiel 6

500 mg Nanodiamanten-Einwaage wird in einer Wasserstoffgasströmung mit einem Durchsatz von 2,5 L/Stunde bei 500° C im Laufe von 5 Stunden ausgeglüht. Die ausgeglühten Nanodiamantenteilchen werden nach dem Verfahren einer Flüssigphasenchlorierung mit molekularem Chlor (5,0 % (Gew.)) in 100 mL CCl₄ im sichtbaren Licht innerhalb von 48 Stunden bei einer Temperatur von 60° C chloriert. Danach wird die Suspension mit einer Geschwindigkeit von 6000 U/min. zentrifugiert und mit trockenem CCl₄ gespült. Der Vorgang wird 5mal wiederholt. Der hergestellte Absatz wird anschließend im Vakuum bis zu einem konstanten Gewicht getrocknet. Die Zielproduktausbeute beträgt 433,5 mg (86,7 %).

Das erzeugte Produkt ist ein graues ultradisperses Pulver mit einer Korngröße von 2 - 10 nm. Der Chloranteil auf seiner Oberfläche beträgt 5,2 % (at.) mit Aggregatgrößen von 63 nm in wässriger Suspension. Das Produkt ist durch folgendes IR-Spektrum gekennzeichnet: ein starker breiter Streifen mit einem Maximum bei 3430 cm⁻¹, ein breiter Streifen mit einem Maximum bei 1262 cm⁻¹, fünf mittelstarke Streifen bei 2929, 2892, 1331, 846, 680 cm⁻¹ und ein schwaches Signal bei 743 cm⁻¹. Die Elementenzusammensetzung der Oberfläche umfasst jeweils C - 86,5, O - 6,1, N - 2,2, Cl - 5,2 % (at.).

### Beispiel 7

500 mg Nanodiamanten-Einwaage wird in einer Wasserstoffgasströmung mit einem Durchsatz von 2,5 L/Stunde bei 1200° C im Laufe von 5 Stunden ausgeglüht. Die ausgeglühten Nanodiamantenteilchen werden nach dem Verfahren einer Flüssigphasenchlorierung mit molekularem Chlor (3,3 % (Gew.)) in 100 mL CCl₄ im sichtbaren Licht innerhalb von 48 Stunden bei einer Temperatur von 60° C chloriert. Danach wird die Suspension mit einer Geschwindigkeit von 7000 U/min. zentrifugiert und mit trockenem CCl₄ gespült. Der Vorgang wird 4mal wiederholt. Der hergestellte Absatz wird anschließend im Vakuum bis zu einem konstanten Gewicht getrocknet. Die Zielproduktausbeute beträgt 370,5 mg (74,1 %).

Das erzeugte Produkt ist ein graues ultradisperses Pulver mit einer Korngröße von 2 - 10 nm. Der Chloranteil auf seiner Oberfläche beträgt 8,8 % (at.) mit Aggregatgrößen von 58 nm in wässriger Suspension. Das Produkt ist durch folgendes IR-Spektrum gekennzeichnet: ein starker breiter Streifen mit einem Maximum bei 3430 cm⁻¹, ein breiter Streifen mit einem Maximum bei 1262 cm⁻¹, fünf mittelstarke Streifen bei 2929, 2892, 1331, 846, 680 cm⁻¹ und ein schwaches Signal bei 743 cm⁻¹. Die Elementenzusammensetzung der Oberfläche umfasst jeweils C - 83,9, O - 5,5, N - 1,8, Cl - 8,8 % (at.).

### Beispiel 8

200 mg Nanodiamanten-Einwaage wird in einer Wasserstoffgasströmung mit einem Durchsatz von 2,0 L/Stunde bei 800° C innerhalb von 1 Stunde ausgeglüht. Die ausgeglühten Nanodiamantenteilchen werden nach dem Verfahren einer Flüssigphasenchlorierung mit molekularem Chlor (4,6 % (Gew.)) in 40 mL CCl₄ im sichtbaren Licht innerhalb von 48 Stunden bei einer Temperatur von 60° C chloriert. Danach wird die Suspension mit einer Geschwindigkeit von 9000 U/min. zentrifugiert und mit trockenem CCl₄ gespült. Der Vorgang wird 3mal wiederholt. Der hergestellte Absatz wird anschließend im Vakuum bis zu einem konstanten Gewicht getrocknet. Die Zielproduktausbeute beträgt 180 mg (90,0 %).

Das erzeugte Produkt ist ein graues ultradisperses Pulver mit einer Korngröße von 2 - 10 nm. Der Chloranteil auf seiner Oberfläche beträgt 3,5 % (at.) mit Aggregatgrößen von 70 nm in wässriger Suspension. Es ist durch folgendes IR-Spektrum gekennzeichnet: ein starker breiter Streifen mit einem Maximum bei 3430 cm⁻¹ ein breiter Streifen mit einem Maximum bei 1262 cm⁻¹, fünf mittelstarke Streifen bei 2929, 2892, 1331, 846, 680 cm⁻¹ und ein schwaches Signal bei 743 cm⁻¹. Die Elementenzusammensetzung der Oberfläche umfasst jeweils C - 87,5, O - 6,9, N - 2,1, Cl - 3,5 % (at.).

### Beispiel 9

300 mg Nanodiamanten-Einwaage wird in einer Wasserstoffgasströmung mit einem Durchsatz von 2,0 L/Stunde bei einer Temperatur von 800° C 8 Stunden lang ausgeglüht. Die ausgeglühten Nanodiamantenteilchen werden nach dem Verfahren einer Flüssigphasenchlorierung mit molekularem Chlor (4,6 % (Gew.)) in 60 mL CCl₄ im sichtbaren Licht innerhalb von 48 Stunden bei einer Temperatur von 60° C chloriert. Danach wird die Suspension mit einer Geschwindigkeit von 6000 U/min. zentrifugiert und mit trockenem CCl₄ gespült. Der Vorgang wird 5mal wiederholt. Der hergestellte Absatz wird anschließend im Vakuum bis zu einem konstanten Gewicht getrocknet. Die Zielproduktausbeute beträgt 256,2 mg (85,4 %).

Das erzeugte Produkt ist ein graues ultradisperses Pulver mit einer Korngröße von 2 - 10 nm. Der Chloranteil auf seiner Oberfläche beträgt 13,2 % (at.) mit Aggregatgrößen von 55 nm in wässriger Suspension. Es ist durch folgendes IR-Spektrum gekennzeichnet: ein starker breiter Streifen mit einem Maximum bei 3430 cm⁻¹ ein breiter Streifen mit einem Maximum bei 1262 cm⁻¹, fünf mittelstarke Streifen bei 2929, 2892, 1331, 846, 680 cm⁻¹ und ein schwaches Signal bei 743 cm⁻¹. Die Elementenzusammensetzung der Oberfläche umfasst jeweils C - 79,8, O - 5,2, N - 1,8, Cl - 13,2 % (at.).

Die Eigenschaften des Systems zur Förderung von bioaktiven Substanzen sowie die Kenndaten des Herstellungsverfahrens dafür in Bezug auf jedes Beispiel sind in Tabelle 2 zusammengefasst.

**Tabelle 2**

| Zusammenfassung der Eigenschaften des neuen Systems zur Förderung von bioaktiven Substanzen und seiner Herstellungsbedingungen | | | | | | | | | |
|---|---|---|---|---|---|---|---|---|---|
| Kenndaten des Vorgangs | Beispiel-Nr. / Vorgangsbedingungen | | | | | | | | |
| | 1 | 2 | 3 | 4 | 5 | 6 | 7 | 8 | 9 |
| Ausglühzeit (1-8), St. | 5 | 5 | 5 | 5 | 5 | 5 | 5 | 1 | 8 |
| Ausglühtemperatur (500-1200), °C | 800 | 800 | 800 | 800 | 800 | 500 | 1200 | 800 | 800 |
| Chlorierungsdauer (36-60), St. | 48 | 36 | 60 | 48 | 48 | 48 | 48 | 48 | 48 |
| Chlorierungstemperatur (50-70), °C | 60 | 60 | 60 | 50 | 70 | 60 | 60 | 60 | 60 |
| Produktausbeute, % | 90,5 | 79,1 | 84,9 | 74,6 | 72,3 | 86,7 | 74,1 | 90,0 | 85,4 |

| | Eigenschaften des Förderungssystems | | | | | | | | |
|---|---|---|---|---|---|---|---|---|---|
| Chlorgehalt, % (at.) | 14 | 4,2 | 7,8 | 3,0 | 9,4 | 5,2 | 8,8 | 3,5 | 13,2 |
| Teilchengröße in der Suspension, nm | 50 | 67 | 56 | 70 | 61 | 63 | 58 | 70 | 55 |

### Beispiel 10

Das angemeldete Förderungssystem wird nach dem Verfahren aus Beispiel 1 hergestellt. 500 mg des Förderungssystems werden in 50 mL des Lösemittels Dimethylsulfoxid aufgeschwemmt, mit 2,5 mL Äthylendiamin ergänzt, zwei Tropfen Pyridin dazugegeben und bei einer Temperatur von 120° C im Laufe von 24 Stunden gehalten. Das hergestellte Konjugat von Nanodiamanten wird dann mit Äthylen-diamin bei einer Geschwindigkeit von 6000 U/min. zentrifugiert, 5mal mit Wasser und Azeton gewaschen und nachher im Vakuum bis zu einem konstanten Gewicht getrocknet.

Das hergestellte Konjugat stellt ein graues ultradisperses Pulver mit einer Korngröße von 2 - 10 nm dar und ist durch ein Raman-Spektrum mit einer starken Lumineszenz gekennzeichnet. Diese Lumineszenz ist mehr als 50-fach stärker als die des P-Spektrums der Nanodiamanten (Fig. 9). Die Elementenzusammensetzung der Oberfläche des hergestellten Konjugats umfasst jeweils C - 86,4, O - 8,9, N - 4,7 % (at.).

Das hergestellte Konjugat wurde zur Förderung von Äthylendiamin in den Körper verwendet.

Um diese Aufgabe zu lösen, wurde bei der Herstellung des Systems zur Förderung von bioaktiven Substanzen, und zwar nach dem Ausglühen der Nanodiamanten, ein Tritiummarker auf die Nanodiamanten nach dem Verfahren einer thermischen Aktivierung durch Tritium aufgetragen [13]. Nach der Behandlung der ausgeglühten Nanodiamanten mit Tritiumatomen wurden sie 48 Stunden im Wasser gehalten, zentrifugiert, vom Überstand getrennt und mit einer neuen Dosiermenge von Löse-mittel ergänzt. Im Endeffekt wurde ein Präparat der ausgeglühten Nanodiamanten mit einer spezifischen Strahlungsaktivität von 90 GBq/g produziert. Danach wird ein Förderungssystem für bioaktive Substanzen nach dem neuen Verfahren hergestellt. Die Oberfläche dieses Systems ist mit einem radioaktiven Marker markiert. Dann wird das Konjugat des Förderungssystems mit Äthylendiamin nach dem oben beschriebenen Verfahren erzeugt. Das hergestellte Konjugat mit dem radioaktiven Marker wird in den Körper einer Ratte (weißes Outbred-Männchen mit einem Körpergewicht von 400 g) in Form von wässriger Suspension intraperitonal eingeführt. 4 Stunden später wird das Tier getötet. Die Organe und Gewebe werden entnommen und gewogen und in Wasserlösungen von NaOH und H₂O₂ homogenisiert. Die Radioaktivität des hergestellten Homogenats wird mit einem Flüssigkeitsszintillationsspektrometer RackBeta 1215 (Finnland) gemessen (Tabelle 3, Fig. 10).

**Tabelle 3**

| Liste der entnommenen Rattenorgane zur Untersuchung der Verteilung des Nanodiamanten-Konjugats mit Äthylendiamin | | | | |
|---|---|---|---|---|
| Probe Nr. | Bezeichnung von Organ bzw. Gewebe | Gewicht, g | NaOH-Menge, mL | H₂O₂-Menge, mL |
| 1 | Leber | 0,42 | 2 | 0,05 |
| 2 | Lungen | 0,13 | 1,5 | - |
| 3 | Milz | 0,26 | 2 | 0,15 |
| 4 | Nieren | 0,22 | 1,5 | 0,05 |
| 5 | Dickdarm | 0,17 | 2 | - |
| 6 | Dünndarm | 0,12 | 1 | - |
| 7 | Herz | 0,27 | 2,05 | 0,05 |
| 8 | Magen | 0,08 | 1,5 | - |
| 9 | Muskeln | 0,1 | 1,5 | - |
| 10 | Blut | 0,27 | 3 | 0,25 |
| 12 | Frontrinde | 0,18 | 1,5 | |
| 13 | Hirnstamm | 0,31 | 2 | 0,05 |
| 15 | Kleinhirn | 0,32 | 2,5 | 0,05 |
| 16 | Nebennieren | 0,08 | 1 | - |

Aus Fig. 10 folgt, dass das Konjugat von Nanodiamanten mit Äthylendiamin so gut wie in allen lebenswichtigen Organen mit verschiedenen Mengenverhältnissen verteilt wird. Dabei wird um die Bluthirnschranke herumgekommen.

### Beispiel 11

200 mg des nach dem im Beispiel 1 beschriebenen Verfahren hergestellten Förderungssystems und 40 mL Alkohol-Wasser-Gemisch (Methanol : Wasser = 1 : 1) werden benutzt, um eine Suspension zu bekommen. Der Suspension werden 300 mg Glyzin in Form von freier Aminosäure NH₂CH₂COOH mit einer Zugabe von 1 mL Triäthylamin zugesetzt. Das hergestellte Gemisch wird mit Ultra-schall (50 W) 40 Minuten lang behandelt und unter ständigem Umrühren und bei einer Temperatur von 65 °C im Laufe von 30 Stunden gehalten. Das erzeugte Produkt wird bei einer Geschwindigkeit von 6000 U/min. zentrifugiert, mit Äthanol gewaschen und dann im Vakuum bei 70° C über die ganze Nacht getrocknet. Die Restfeuchte des Produkts beträgt 2,2 %. Die Zielproduktausbeute beträgt 186 mg (93 %).

Das erzeugte Produkt stellt ein dunkelgraues, blau angefärbtes ultradisperses Pulver mit Primärteilchengrößen im Bereich von 2 - 10 nm dar. Die Teilchen haben eine bis 1 nm starke Umhüllung einer Oberflächenschicht (Fig. 11). Das Produkt ist durch folgendes IR-Spektrum gekennzeichnet: ein starker breiter Streifen mit einem Maximum bei 3400 cm⁻¹, ein starkes Signal bei der Frequenz von 1621 cm⁻¹, sechs mittelstarke Streifen bei 2924, 2881, 1383, 1306, 1212 und 1154 cm⁻¹ und ein schwaches Kennsignal bei 504 cm⁻¹ (Fig. 12). Die Elementenzusammensetzung der Konjugatoberfläche umfasst jeweils: C - 91,5, O - 6,0, N - 2,5 % (at.).

Das hergestellte Konjugat wurde zur Förderung von Glyzin in den Körper verwendet. Die Eindringung des Nanodiamanten-Konjugats mit Glyzin in den Körper wird durch Forschungen der Zusammenwirkung zwischen dem Nanodiamanten-Konjugat mit Glyzin und der Lymphoblast-Zellkultur MOLT-4 mit Hilfe eines elektronischen Mikroskops nachgewiesen. Aus Fig. 13 ist ersichtlich, dass unter der Konjugatwirkung Invaginationen der Zellmembran der Lymphoblastzelle sowie eine weitere Eindringung ins Zytosol erfolgen.

### Schriftennachweis

1. Patent RU 2391966, C1. 20.06.2010.
2. Nano-Heilmittel. Konzepte über die Heilmittelförderung in der Nano-Wissenschaft: Übersetzung aus dem Englischen. / Herausgegeben von Alf Lamprecht, M.: Nautchny Mir, 2010. S. 10-20.
3. J.L. Grangier, M. Puygrenier, J.C. Gautier, P. Couvreur. Nanoparticles as carriers for growth hormone releasing factors // J. Control. Rel. 1991. V.15. P. 3-13.
4. A. Adnant, R. Lam, H. Chen et al. Atomistic Simulation and Measurement of pH Dependent Cancer Therapeutic Interactions with Nanodiamond Carrier // Mol. Pharmaceutics. 2001. V. 8.. P. 368-374.
5. K.-K. Liy, W.-W. Zheng, C.-C. Wang et al. Covalent linkage of nanodiamondpaclitaxel for drug delivery and cancer therapy // Nanotechnology. 2010. V. 21. JY° 315106. 14 pp.
6. Patent US 2005/0158549 A1, 21.07.2005.
7. Patent US 2010/0129457 A1, 27.05.2010.
8. Russisches Sammelbuch für Arbeitsschutz. 3 Bände. 2. aktualisierte Auflage. Band 3. M.: Verlag NZ ENAS. 2007. S. 181.
9. N.N. Karkischschenko. Heilmittel aus dem Bereich Nanoengineering: Neue biomedizinische Initiativen in Pharmakologie // Biomedizina. 2009. Heft 2. S. 5-26.
10. Patent US 2009/0226495 A1, 10.09.2009.
11. G.V. Lisichkin, I.I. Kulakova, Y.A. Gerasimov et al. Halogenation of detonationsynthesised nanodiamond surfaces. Mendeleev Commun. 2009. V. 19. P. 309-310.
12. A. Smith. Angewandte IR-Spektroskopie. Übersetzung aus dem Englischen - M.: Mir, 1982. S. 307.
13. G. A. Badun. Tritiummarkierte Verbindungen / Verfahren, Anleitung. - M.: MGU, 2008. S. 36-37.

## Patentansprüche

1. System zur Förderung der Aufnahme von bioaktiven Substanzen in den Körper in Form von einem Nanodiamanten mit einer Korngröße von 2 - 10 nm, wobei die Teilchenoberfläche mit Chlor bei einem Chlorgehalt bis zu 14 % (at.) modifiziert ist.

2. Verfahren zur Herstellung von einem System zur Förderung der Aufnahme von bioaktiven Substanzen in den Körper nach Anspruch 1,
**dadurch gekennzeichnet,**
**dass** Nanodiamantenteilchen bei einer Temperatur von 500 - 1200° C in einer Wasserstoffgasströmung ausgeglüht werden und
**dass** diese Teilchen danach mit dem in Tetrachlorkohlenstoff aufgelösten molekularen Chlor unter photochemischer Wirkung des sichtbaren Lichts und bei einer Temperatur von 50 - 70° C nach dem Verfahren einer Flüssigphasenchlorierung chloriert werden.

3. Verfahren nach Anspruch 2,
**dadurch gekennzeichnet,**
**dass** das Ausglühen der Ausgangs-Nanodiamanten mit einem Wasserstoffgasdurchsatz von 2 bis 3 L/Stunde durchgeführt wird.

4. Verfahren nach Anspruch 2,
**dadurch gekennzeichnet,**
**dass** die Chlorierung im Laufe von 36 bis 60 Stunden bei einer molekularen Chlorkonzentration von 3 bis 5 % (Gew.) in Tetrachlorkohlenstoff durchgeführt wird, und dass anschließend eine Zentrifugierung, eine Waschung mit Tetrachlorkohlenstoff und eine Trocknung vorgenommen werden.

5. Nanodiamant mit einer Korngröße von 2-10 nm, wobei die Teilchenoberfläche mit Chlor bei einem Chlorgehalt bis zu 14 % (at.) modifiziert ist, hergestellt mit einem Verfahren nach einem oder mehreren der Ansprüche 2 bis 4.

## Claims

1. System for promoting the uptake of bioactive substances into the body, in the form of a nanodiamond having a grain size of 2-10 nm, wherein the particle surface is modified with chlorine at a chlorine content up to 14% (at.).

2. Method for producing a system for promoting the uptake of bioactive substances into the body according to Claim 1,
**characterized in that**
nanodiamond particles are annealed in a hydrogen gas flow at a temperature of 500-1200°C and
**in that** these particles are thereafter chlorinated by the method of liquid-phase chlorination using the molecular chlorine dissolved in carbon tetrachloride under photochemical action of visible light and at a temperature of 50-70°C.

3. Method according to Claim 2,
**characterized in that**
the starting nanodiamond is annealed at a hydrogen gas throughput of 2 to 3 l/hour.

4. Method according to Claim 2,
**characterized in that**
the chlorination is carried out in the course of 36 to 60 hours at a molecular chlorine concentration of 3 to 5% (by weight) in carbon tetrachloride, and that then a centrifugation, a washing with carbon tetrachloride and a drying are performed.

5. Nanodiamond having a grain size of 2-10 nm, where the particle surface is modified with chlorine at a chlorine content up to 14% (at.), produced by a method according to one or more of Claims 2 to 4.

## Revendications

1. Système pour faciliter l'absorption de substances bioactives dans le corps, sous forme d'un nanodiamant ayant une taille de particule de 2 - 10 nm, la surface des particules étant modifiée avec du chlore, à une teneur en chlore de jusqu'à 14 % (en atomes).

2. Procédé pour la préparation d'un système destiné à faciliter l'absorption de substances bioactives dans le corps selon la revendication 1,
**caractérisé en ce**
**qu'**on soumet les particules de nanodiamant à un recuit à une température de 500 - 1 200 °C dans un courant d'hydrogène gazeux et
**qu'**on soumet ensuite ces particules à une chloration avec le chlore moléculaire dissous dans du tétrachlorure de carbone sous l'action photochimique de la lumière visible et à une température de 50 - 70 °C selon le procédé d'une chloration en phase liquide.

3. Procédé selon la revendication 2,
**caractérisé en ce**
**qu'**on effectue le recuit des nanodiamants de départ à un débit d'hydrogène gazeux de 2 à 3 litres/heure.

4. Procédé selon la revendication 2,
**caractérisé en ce**
**qu'**on effectue la chloration en l'espace de 36 à 60 heures à une concentration de chlore moléculaire de 3 à 5 % (en poids) dans du tétrachlorure de carbone, et en ce qu'on effectue ensuite une centrifugation, un lavage avec du tétrachlorure de carbone et un séchage.

5. Nanodiamant ayant une taille de particule de 2 - 10 nm, la surface des particules étant modifiée avec du chlore, à une teneur en chlore de jusqu'à 14 % (en atomes), produit par un procédé selon une ou plusieurs des revendications 2 à 4.
